# EUROPEAN PATENT APPLICATION

(11) **EP 4 763 234 A1**
(43) Date of publication of application: **24.06.2026**
(21) Application number: 25204623.0
(22) Date of filing: 25.09.2025
(51) Int. Cl.: A61L 12/04, B65B 55/14, C08L 29/04, C08L 39/06, G02B 1/04, G02C 7/04

(54) **CONTACT LENS PACKAGING SOLUTION AND METHOD FOR MANUFACTURING CONTACT LENS**

(30) Priority: 17.12.2024 TW 113149200
(71) Applicant: Vizionfocus Inc, Zhunan Township, Miaoli County 35059 (TW)
(72) Inventor: HSU, Wei-Hang, 351002 Toufen City, Miaoli County (TW); YU, Chu-Yi, 351011 Toufen City, Miaoli County (TW); CHENG, Yao-Tsung, 852006 Kaohsiung City (TW); HSU, Hui-Wen, 351011 Toufen City, Miaoli County (TW)
(74) Representative: Zacco Denmark A/S

(57) **Abstract**

The present invention provides a contact lens packaging solution, suitable for soaking a core lens body, and performing a sterilization step when the core lens body is soaked in the contact lens packaging solution. The contact lens packaging solution includes a first polymer and a second polymer. The first polymer comprises polyvinyl alcohol, and the second polymer comprises polyvinylpyrrolidone. The present invention also provides a method for manufacturing contact lenses.

## Description

### CROSS-REFERENCE TO RELATED APPLICATION

This application claims the priority benefit of Taiwan Application No. 113149200, filed on December 17, 2024. The entirety of the above-mentioned patent application is hereby incorporated by reference herein and made a part of this specification.

### FIELD OF THE INVENTION

The present invention relates to a contact lens packaging solution and a method for manufacturing contact lens.

### BACKGROUND OF THE INVENTION

A soft contact lens material has been introduced from 1960s'. Since the material is soft, the discomfort brought by an original rigid gas-permeable lens (RGP Lens) in wearing has been overcome. A user who is originally not adapted to the rigid gas-permeable lens may use the soft contact lens. The soft contact lens, thus, becomes a main option for the contact lens user now.

Compared with the rigid gas-permeable lens, although the soft contact lens has been much soft and comfortable, still many users reflect that they feel something in eyes when wearing the soft contact lens, which prompts the users to abandon the contact lens. The primary reason that causes the above problem lies in that the surface of the contact lens has a frictional force. Therefore, when the user blinks, the lens moves on the eyeball and rubs with the eyeball, resulting in that the user feels that there is something in the eyes. Therefore, to increase the surface lubricity of the lens may solve the above problem.

It has been shown by many research inputs that after the surface of the lens has been covered with polyacrylic acid (PAA), a surface lubrication effect has been achieved. However, PAA is an anionic polymer which tends to have negative charges, and its property is easily affected by the pH value of tear, which may result in an irritant problem in wearing. Subsequent researches disclose neutralization of the electrical property of PAA by a cationic polymer, which aims to improve the stability of the above technology. However, in such a manner, a lot of control cost has to be input in the manufacturing process, and the product is unable to maintain the lubrication effect when the contact lens is worn for a long time.

### SUMMARY OF THE INVENTION

In view of the above problem and technical development, the present invention provides a method capable of effectively improving surface lubricity of a contact lens. The effect can be maintained for a long time, and moreover, a technical solution with cost competitiveness is provided.

Unless otherwise defined, all technical and scientific terms used herein have the same meaning as commonly understood by those skilled in the art. Generally, the nomenclature and the laboratory procedure used herein are known in the art and usually used. Conventional methods are used in the processes, for example, methods mentioned in the art and various common references. When the terms are provided in singular forms, the inventor also considers the plural forms of the terms. The nomenclature used herein and the laboratory procedure described below are known in the art and usually used. As used in the entire disclosure, unless otherwise specified, the following terms shall be understood as having the following meaning.

A dry lens refers to a lens which has been subjected to curing but has not yet been subjected to hydration during a contact lens production process.

A wet lens refers to a lens which has been subjected to hydration during a contact lens production process.

A contact lens packaging solution refers to an aqueous solution that undergoes a sterilization process along with a wet lens during a contact lens production process. The contact lens formed through the sterilization process is usually continued to be preserved in the aqueous solution, so the aqueous solution is also called a contact lens packaging solution. In addition, the aqueous solution usually has a buffering function, that is, has a pH value and an osmotic pressure within a certain range. Therefore, buffer is also used herein to refer to a contact lens packaging solution.

A core lens body refers to a portion of a lens that is formed from a contact lens formulation after subjected to curing. The core lens body can be a dry lens or a wet lens. Part of implemented samples listed in the present invention does not have the shell layer.

After the core lens body has been formed, a shell layer is then formed on the surface of the core lens body in other processes after curing. But the shell layer is not a structure of the core lens body which has been originally formed. Moreover, by forming a stable structure similar to an interpenetrating polymer network, the shell layer may stably exist on the surface of the core lens body.

The interpenetrating polymer networks (IPN): the IPN is usually defined as including two or more polymers theoretically. At least one polymer is a crosslinked polymer network. Moreover, at least two or more polymers are partially staggered in polymer scale without a covalent bond formed. For example, a polymer A itself is a crosslinked polymer network, and a polymer B is further partially staggered with the polymer A. The covalent bond is not formed between the polymer A and the polymer B. Therefore, the polymer A and the polymer B form the IPN. In view of polymers and complexity of field of chemistry, objects indicated by the IPNs used in the present invention, that are, different polymers allow covalent bonding to happen in the process and result of forming the IPNs. For example, in the case disclosed by the present invention, there are a small amount (less than 0.1wt%) of the first polymer and the polymer of the core lens body subjected to covalent bonding, which is not excluded. To facilitate expression, the inventor still uses IPN to describe the partially staggered polymer network formed by the first polymer on the surface of core lens body and the polymer of the core lens body with the IPN. The shell layer is a polymer structure formed by entanglement of the first polymer and the second polymer and is called as the stable structure similar to the IPN. In the polymer structure, the polymeric chain of the first polymer itself does not have obvious crosslinking, so does the second polymer itself. There are strong hydrogen bonds between the polymeric chains of the first polymer. Moreover, there are still strong hydrogen bonds between the polymeric chains of the first polymer and the second polymer, thereby forming the stable structure similar to the IPN. Thus, the network structure formed by the first polymer and the second polymer in the shell layer is not directly described with the IPN to approach to a generally theoretical definition as far as possible.

Semi interpenetrating polymer structure (Semi-IPN): a generally theoretical definition means that at least one polymer is a linear polymer in the IPN. The IPN in the present invention may include Semi-IPN.

A simultaneously interpenetrating polymer network (Simultaneous-IPN): a generally theoretical definition means that formation of the IPN is originated from the polymerization reaction of the constitutional units of two or more polymers. For example, an IPN is composed of a polymer Γ and a polymer Δ. The polymer Γ is formed by a monomer γ through a polymerization reaction, and the polymer Δ is formed by a monomer δ through a polymerization reaction. If the monomer γ and the monomer δ are mixed and the mixture is subjected to the polymerization reaction, the finally formed IPN of the polymer Γ and the polymer Δ may be the Simultaneous-IPN. The IPN involved in the present invention does not include, for example, the Simultaneous-IPN.

The present invention provides a contact lens packaging solution, suitable for soaking a core lens body, and performing a sterilization step when the core lens body is soaked in the contact lens packaging solution. The contact lens packaging solution includes a first polymer and a second polymer. The first polymer includes polyvinyl alcohol, and the second polymer includes polyvinylpyrrolidone.

The present invention provides a contact lens, including a core lens body and a shell layer, wherein the shell layer covers the core lens body. The shell layer includes at least two polymers: a first polymer and a second polymer.

The first polymer used in the present invention is a polymer including a polyvinyl alcohol (PVA) structure. The first polymer may be polyvinyl alcohol or a copolymer of polyvinyl alcohol and polyvinyl acetate (PVA-PVAc copolymer) or a mixture thereof. The first polymer may be cross-linkable or uncross-linkable. The copolymer of polyvinyl alcohol and polyvinyl acetate may be a block copolymer, an alternative copolymer, a random copolymer, or a graft copolymer. Polyvinyl alcohol may be cross-linkable or uncross-linkable. Polyvinyl alcohol may be fully hydrolyzed, that is, fully hydrolyzed PVA, or may be partially hydrolyzed, that is, partially hydrolyzed PVA. The partially hydrolyzed PVA is preferred. In terms of classification, the partially hydrolyzed PVA may also be understood as the PVA-PVAc copolymer because the polymer chain segment of the partially hydrolyzed PVA has two units: both vinyl alcohol and vinyl acetate. Furthermore, the fully hydrolyzed PVA is polyvinyl alcohol with 100% vinyl alcohol units. The partially hydrolyzed PVA, such as a partially hydrolyzed polyvinyl alcohol with a degree of hydrolysis of 80%, means that it contains 80% vinyl alcohol units and 20% unhydrolyzed vinyl acetate units. In terms of lexical representation of the present invention, the partially hydrolyzed polyvinyl alcohol and the copolymer of polyvinyl alcohol and polyvinyl acetate may refer to the same objects. The two terms may be interchangeably used.

In the present invention, the first polymer is preferably the partially hydrolyzed PVA. A preferred range of a degree of hydrolysis of the partially hydrolyzed PVA is 70%~99%, the more preferred range is 80%~98%, and the more preferred range is 88%~95%. It is found in the present invention that the proper degree of hydrolysis of polyvinyl alcohol may increase the stability of the IPN formed by the polyvinyl alcohol and the polymer on the surface of the core lens body, and may also increase the stability of the IPN formed by the first polymer and the second polymer in the shell layer. The reason that the partially hydrolyzed polyvinyl alcohol may be superior to the fully hydrolyzed polyvinyl alcohol lies in that the intramolecular hydrogen bonding of the fully hydrolyzed polyvinyl alcohol is stronger, easily resulting in that the fully hydrolyzed polyvinyl alcohol is less likely to enter the surface structure of the core lens body (polymer), so that the IPN is unlikely formed by the fully hydrolyzed polyvinyl alcohol and the core lens body (polymer); similarly, due to the stronger intramolecular hydrogen bonding, the fully hydrolyzed polyvinyl alcohol is less likely to form intermolecular hydrogen bonds with the second polymer containing polyvinylpyrrolidone. Therefore, compared with the partially hydrolyzed polyvinyl alcohol, the fully hydrolyzed polyvinyl alcohol is less likely to form the similar IPN with the second polymer.

A preferred range of the molecular weight (Mw) of the first polymer is 5,000 Da~300,000 Da, more preferably in the range of 10,000 Da~200,000 Da, more preferably in the range of 15,000 Da~150,000 Da, and more preferably in the range of 25,000 Da~100,000 Da.

The second polymer used in the present invention is a polymer including a polyvinyl pyrrolidone structure. The second polymer may be polyvinylpyrrolidone, a copolymer including polyvinylpyrrolidone, or a mixture of the two. The second polymer may be cross-linkable or uncross-linkable. The copolymer including polyvinylpyrrolidone may be a copolymer of vinylpyrrolidone and dimethylaminoethyl methacrylate, also called Poly(vinylpyrrolidone)-co-(dimethylaminoethylmethacrylate). A specific example may be a product sold by Ashland Inc. such as Copolymer 845, Copolymer 937 or Copolymer 958. The molecular weight of the copolymer of vinylpyrrolidone and dimethylaminoethyl methacrylate is, for example, greater than or equal to 100,000 Da. The preferable molecular weight of the copolymer of vinylpyrrolidone and dimethylaminoethyl methacrylate is, for example, greater than or equal to 1,000,000 Da.

A preferred range of the molecular weight (Mw) of the second polymer is greater than or equal to 8,000 Da, more preferred range is greater than or equal to 100,000 Da, more preferred range is greater than or equal to 160,000 Da, and more preferred range is greater than or equal to 360,000 Da. A relatively effective shell layer may be formed within the above-mentioned molecular weight of the second polymer. When the molecular weight of the second polymer is not great enough, it is hardly to form the effective shell layer with the first polymer. It is speculated that when the molecular weight of the second polymer is not great enough, although the second polymer still can form similar IPN with the first polymer, the second polymer is prone to moving in the structure due to its small molecular weight, resulting in that the shell layer is insufficient in stability.

At the junction of the core lens body and the shell layer, the IPN is formed by the first polymer and the polymer structure of the core lens body. In most area of the shell layer, the stable structure similar to the IPN is formed between the first polymer and the second polymer.

At the junction of the core lens body and the shell layer, there is the IPN formed by the first polymer and the polymer structure of the core lens body. In the IPN, a part of first polymers is freely movable polymers while stable bonding is formed among the other part of the first polymers. The bonding makes the IPN stable. Formation of the bonding is mainly originated from an autoclaving process of the contact lens. This is because stable hydrogen bonds are generated among polyvinyl alcohol due to high temperature and high pressure in the autoclaving process. Therefore, the part of the shell layer in contact with the core lens body mainly includes the first polymer and the part of the shell layer away from the core lens body mainly includes the second polymer. In most area of the shell layer, the stable structure similar to the IPN is formed between the first polymer and the second polymer. A part of the first polymers is freely movable polymers, a part of the second polymers is freely movable polymers. Stable bonds are formed among part of the first polymers, and the bonding makes the similar IPN stable. Formation of the bonding is mainly originated from the stable hydrogen bonds generated between polyvinyl alcohol and polyvinyl alcohol during an autoclaving process of the contact lens. Moreover, stable hydrogen bonds may also be generated between polyvinyl alcohol and polyvinylpyrrolidone, which are mainly originated from hydroxyl groups on polyvinyl alcohol and oxygen atoms on polyvinylpyrrolidone. Therefore, the entire shell layer may be relatively stable in structure, thereby maintaining the comfort of the user in the wearing process.

For further description, the proportion of vinyl acetate in the first polymer (i.e. the degree of hydrolysis of the partially hydrolyzed polyvinyl alcohol) may obviously affect the effect of forming the shell layer. The polyvinyl alcohol structure itself has strong crystallinity. If the crystallinity of the first polymer is too strong, before the shell layer is formed, the first polymer cannot effectively enter the surface structure of the core lens body, resulting in that the first polymer cannot form the semi-IPN with the surface structure of the core lens body, and accordingly, the relatively stable IPN cannot be formed in the subsequent autoclaving process. Moreover, if the crystallinity of the first polymer is too strong, oxygen atoms on polyvinylpyrrolidone of the second polymer hardly form the hydrogen bonds with the hydroxyl groups of polyvinyl alcohol of the first polymer. Therefore, it is unlikely to generate enough entanglement between the first polymer and the second polymer, which will also affect the effect of forming the similar IPN by the first polymer and the second polymer, and further affects the stability of the shell layer. Moreover, a part of vinyl acetate in the first polymer may be reacted with a part of vinyl alcohol during sterilization to undergo a similar crosslinking reaction, so that the structure of the shell layer may further be stable, assisting the shell layer to fix and cover the core lens body.

An important reason to form the stable shell layer mentioned in the present invention is that the first polymer can form the IPN with the core lens body, and the first polymer also can form the IPN with the second polymer. An important reason to form the stable IPN is the autoclaving process. This is because stable hydrogen bonds are generated among polyvinyl alcohol in the first polymer due to high temperature and high pressure during autoclaving process. Moreover, a part of vinyl acetate in the first polymer may be reacted with a part of vinyl alcohol during autoclaving process to undergo a similar crosslinking reaction. The original surface structure of the first polymer and the core lens body which is the originally formed semi-IPN is converted into the relatively stable IPN. In a similar manner, originally, the first polymer and the second polymer in the shell layer are merely subjected to pure physical adsorption or less entanglement. Subjected to high temperature and high pressure during sterilization, the hydrogen bonds and reactions similar to the crosslinking reaction are formed among the first polymers, and moreover, the hydrogen bonds between the first polymer and the second polymer are also enhanced, so that the strength of the entanglement is further strengthened. Thus, the autoclaving process not only helps the shell layer stably cover the core lens body, but also helps enhance the stability of the shell layer itself.

The first polymer used in the present invention may neither include the cationic polymeric segment nor the anionic polymeric segment.

The first polymer used in the present invention may include a cationic polymeric segment. The cationic polymeric segment shall neither generate an inhibitory effect to the IPNs formed by the first polymer and the polymer of the core lens body nor generate an inhibitory effect on stable entanglement formed by the first polymer and the second polymer. Similarly, the first polymer used in the present invention may include an anionic polymeric segment. The anionic polymeric segment shall neither generate an inhibitory effect to the IPNs formed by the first polymer and the polymer of the core lens body nor generate an inhibitory effect on stable entanglement formed by the first polymer and the second polymer.

The second polymer used in the present invention may neither include the cationic polymeric segment nor the anionic polymeric segment.

The second polymer used in the present invention may include a cationic polymeric segment. The cationic polymeric segment shall neither generate an inhibitory effect to the IPNs formed by the first polymer and the polymer of the core lens body nor generate an inhibitory effect on stable entanglement formed by the second polymer and the first polymer. Similarly, the second polymer used in the present invention may include an anionic polymeric segment. The anionic polymeric segment shall neither generate an inhibitory effect to the IPNs formed by the first polymer and the polymer of the core lens body nor generate an inhibitory effect on stable entanglement formed by the second polymer and the first polymer.

The present invention also provides a method for manufacturing a contact lens using the above-mentioned contact lens packaging solution.

In the present invention, the shell layer primarily comes from the first polymer and the second polymer in the contact lens packaging solution, and the shell layer is formed during the sterilization process.

In the present invention, the relative proportion by weight percentage of the first polymer to the second polymer in the contact lens packaging solution may be between 2:1 and 1:15, preferably between 1:2 and 1:10, and more preferably between 1:4 and 1:8. In the selected relative proportion range of the first polymer to the second polymer, the shell layer is formed more stably and is less likely to disappear over time after formation.

In the present invention, the relative proportions by weight percentage of the first polymer to the second polymer in the contact lens packaging solution may be relevant to the molecular weight of the second polymer, and a more stable shell layer may be formed when the two have a better combination. A preferable combination is that the relative proportion by weight percentage of the first polymer to the second polymer in the contact lens packaging solution is between 1:2 and 1:10, and the molecular weight of the second polymer is 160,000 Da or more. A more preferable combination is that the relative proportion by weight percentage of the first polymer to the second polymer in the contact lens packaging solution is between 1:4 and 1:8, and the molecular weight of the second polymer is 360,000 Da or more.

In the present invention, the relative proportion by weight percentage of the first polymer to the second polymer in the contact lens packaging solution may be relevant to the degree of hydrolysis of the first polymer, and a more stable shell layer may be formed when the two have a better combination. A preferable combination is that the relative proportion by weight percentage of the first polymer to the second polymer in the contact lens packaging solution is between 1:2 and 1:10, and the first polymer is partially hydrolyzed polyvinyl alcohol with a degree of hydrolysis between 80% and 98%. A more preferable combination is that the relative proportion by weight percentage of the first polymer to the second polymer in the contact lens packaging solution is between 1:4 and 1:8, and the first polymer is partially hydrolyzed polyvinyl alcohol with a degree of hydrolysis between 88% and 95%.

In the present invention, the contact lens packaging solution has a range of pH value between 6.0 and 8.0, and an osmotic pressure ranging from 200 to 400 mOsm/ (kg H₂O).

To facilitate the reader to read and understand, the structural relationship and principle between the first polymer and the core lens body and the structural relationship and principle between the first polymer and the second polymer are described herein to the utmost with a detail theory. The theoretical description shall not be extended for being further interpreted as a limitation to the scope of the claims of the present invention.

As shown in the embodiment, according to the contact lens provided by the present invention, the core lens body may mainly contains HEMA (2-hydroxyethyl methacrylate), that is, HEMA accounts for over 85% of the contact lens, or HEMA accounts for over 90% of the contact lens, or HEMA accounts for over 95% of the contact lens. Calculation of the composition and proportion herein does not include a diluent. This type of contact lens is also regarded as a hydrogel contact lens.

As shown in the embodiment, according to the contact lens provided by the present invention, the core lens body may be the contact lens including a silicone containing component. The silicone containing component may be a silicone monomer or a silicone macromer, and the silicone containing component may also include a silicone crosslinker. This type of contact lens is also regarded as a silicone hydrogel contact lens.

Other objectives, features and advantages of the invention will be further understood from the further technological features disclosed by the embodiments of the invention wherein there are shown and described preferred embodiments of this invention, simply by way of illustration of modes best suited to carry out the invention.

### BRIEF DESCRIPTION OF THE DRAWINGS

FIG. 1 is a cross-sectional schematic diagram of a contact lens placed in a contact lens package according to an embodiment of the present invention;
FIG. 2 is an enlarged schematic diagram of a circle A in FIG. 1; and
FIG. 3 is a schematic flow diagram of a method for manufacturing a contact lens according to an embodiment of the present invention.

### DETAILED DESCRIPTION OF PREFERRED EMBODIMENTS

Embodiments of the present invention will be referred in detail now, and one or more examples will be illustrated. Each embodiment is provided only for explaining the present invention, rather than limiting the scope of the present invention. Actually, it is apparent to those skilled in the art that modifications and variations on the present invention may be made without departing from the scope or the spirit of the present invention. For example, features depicted or described as a part of an embodiment may be used in another embodiment to generate yet another new embodiment. Thus, the present invention is intended to cover this kind of modifications and variations classified into the scope of the attached claims and equivalents thereof. Other objects, features, and directions of the present invention are disclosed in the detailed description below or are apparent in the detailed description below. Those skilled in the art shall understand that current discussion is merely description of the exemplary embodiment, rather than limitation of wider aspects of the present invention.

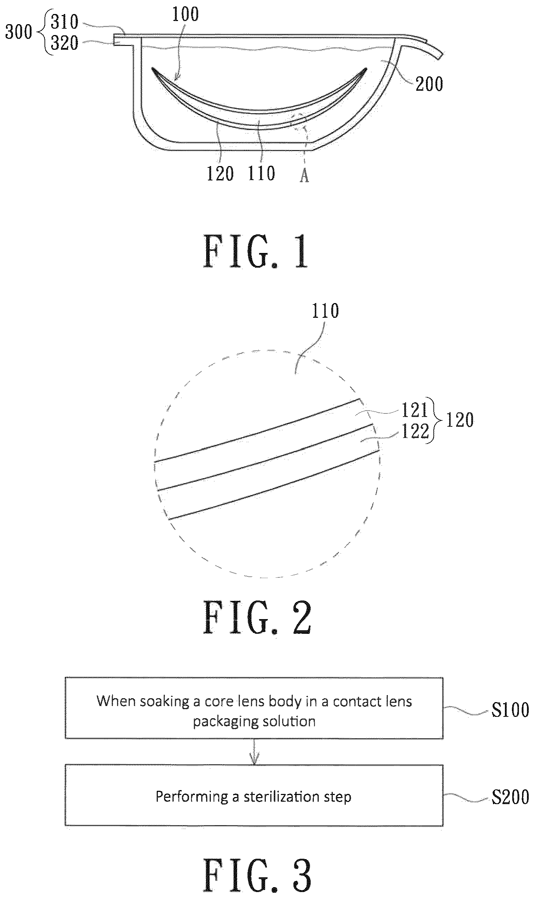

FIG. 1 is a cross-sectional schematic diagram of a contact lens placed in a contact lens package according to an embodiment of the present invention. FIG. 2 is an enlarged schematic diagram of a circle A in FIG. 1. FIG. 3 is a schematic flow diagram of a method for manufacturing a contact lens according to an embodiment of the present invention. Please refer to FIG. 1 and FIG. 3 first. In an embodiment of the present invention, a contact lens packaging solution 200 is suitable for soaking a core lens body 110; and when the core lens body 110 is soaked in the contact lens packaging solution 200, as the step S100 shown in FIG. 3, a sterilization step performs, as the step S200 shown in FIG. 3. The contact lens packaging solution 200 includes a first polymer and a second polymer. The first polymer includes polyvinyl alcohol, and the second polymer includes polyvinyl pyrrolidone. Referring to FIG. 2. a contact lens 100 in an embodiment of the present invention includes a core lens body 110 and a shell layer 120, wherein the shell layer 120 covers the core lens body 110. The shell layer 120 includes a first polymer 121 and a second polymer 122. The first polymer 121 includes polyvinyl alcohol, and the second polymer 122 includes polyvinylpyrrolidone. The first polymer 121 covers, for example, the core lens body 110, and the second polymer 122 covers, for example, the first polymer 121.

Specifically, a contact lens 100 in an embodiment of the present invention is, for example, placed in a contact lens package 300. The contact lens package 300 contains a contact lens packaging solution 200, and the contact lens 100 is soaked in the contact lens packaging solution 200. The contact lens package 300 in this embodiment includes, for example, an aluminum foil 310 and a container 320, where the container 320 has a cavity suitable for containing a contact lens 100 and the contact lens packaging solution 200, and the container 320 is, for example, sealed with the aluminum foil 310, but the present invention is not specifically limited thereto. In this embodiment, the container 320 is, for example, a container made of polypropylene (PP) as a material (also referred to as a "PP cup" herein), but this embodiment is not limited thereto as well.

Component codes and compound descriptions of a contact lens formulation:

| Component abbreviations of the contact lens formulation | full names of the component of the contact lens formulation | Component codes of the contact lens formulation |
|---|---|---|
| TRIS | 3-[Tris(trimethylsilyloxy)silyl]propyl Methacrylate | CAS 17096-07-0 |
| MPDMS | monoMethacryloxypropyl terminated Polydimethylsiloxane | CAS 146632-07-0 |
| NVP | N-Vinylpyrrolidone | CAS 88-12-0 |
| HEMA | 2-Hydroxyethyl Methacrylate | CAS 868-77-9 |
| MAA | Methacrylic Acid | CAS 79-41-4 |
| EGDMA | Ethylene Glycol Dimethacrylate | CAS 97-90-5 |
| Irgracure 819 | Phenylbis(2,4,6-trimethylbenzoyl)phosphine oxide | CAS 162881-26-7 |
| Glycerin | | CAS 56-81-5 |
| 1-Hexanol | | CAS 111-27-3 |
| DEGBE | Diethylene Glycol Monobutyl Ether | CAS 112-34-5 |

An implemented sample 1-1(EX1-1): manufacture a Hydrogel-1 dry lens, with the contact lens formulation shown in Table 1. A process of manufacturing the dry lens from the contact lens formulation is a known technology in the art, and the dry lens may be manufactured by a casting method. A curing mode of the contact lens may be photocuring or thermal curing, and in the implemented sample, photocuring is used. The dry lens is hydrated with 70±5 degrees Celsius hot water (reverse osmosis (RO) water is used) for 60 min, and residual monomer, crosslinker, initiator, and diluent in the dry lens after the curing reaction are cleaned to obtain a wet lens. After the wet lens is obtained, the wet lens is soaked in an aqueous solution containing 0.2 g/L PVA-PVAc copolymer for 120 min to make the PVA-PVAc copolymer enter the surface layer of the core lens body through mass transfer. The PVA-PVAc copolymer used herein is PVA-PVAc-1 with the molecular weight of 31,000 Da and PVA accounting for overall proportion of 88%, that is, partially hydrolyzed polyvinyl alcohol with a degree of hydrolysis being 88%. After the above steps are finished, the wet lens is moved out of the aqueous solution containing the PVA-PVAc copolymer and placed in a polypropylene cup (pp cup). A buffer (Buffer-1) is dropwise added into the pp cup, and the liquid level of the buffer must be higher than the wet lens to ensure that buffer covers all the wet lens. After the wet lens is covered with the buffer, using heat sealing to seal the aluminum foil with the pp cup, and the wet lens is sent into an autoclave for autoclaving. A sterilization condition is as follows: the temperature is continuously raised to 122 degrees Celsius, and the temperature of 122 degrees Celsius at two barometric pressures is maintained for 30 min, in order to obtain the contact lens, wherein the core lens body takes HEMA as a main component, the water content is about 38%, the shell layer is based on PVA-PVAc copolymer and PVP, and the core lens body and the PVP are connected through the PVA-PVAc copolymer. In about 12 hours (including the sterilization process) after the wet lens is in contact with the buffer, an aluminum foil is torn apart, the buffer was extracted and tested, obtaining a pH value being 7.2 and an osmotic pressure range being 313 mOsm/ (kg H₂O); and the contact lens is taken out and placed in a standard buffer for balancing. One contact lens uses 10 ml of standard buffer. The buffer is replaced every 1 hour, the operation is repeated 3 times, and then the surface lubricity of the contact lens is scored. A formulation proportion (also called "formulation" for short) of the Buffer-1 and the Standard buffer is shown in Table 2.

**Table 1**

| Contact Lens Formulation | | | |
|---|---|---|---|
| | Hydrogel-1 | Hydrogel-2 | Silicone Hydrogel |
| TRIS | | | 22.0% |
| MPDMS | | | 20.0% |
| NVP | | | 41.0% |
| HEMA | 98.5% | 96.7% | 14.0% |
| MAA | 0.1% | 2.0% | |
| EGDMA | 1.0% | 1.0% | 2.0% |
| Irgracure 819 | 0.4% | 0.3% | 1.0% |
| SUM (Not including the diluent) | 100.0% | 100.0% | 100.0% |
| | | | |
| Glycerin | 15.0% | 15.0% | |
| 1-Hexanol | | | 10.0% |
| DEGBE | | | 10.0% |

Annotation: The diluent (including Glycerin, 1-Hexanol, and DEGBE) in the Table 1 is not calculated in the sum (SUM) of the contact lens formulation. The diluent is further additionally added according to 100% SUM as the denominator. For example, if the Hydrogel-1 formulation contains HEMA 98.5g, MAA 0.1g, EGDMA 1.0g, and Irgracure 819 0.4g and the total weight is 100g, Glycerin 15.0g is additionally added as the diluent.

**Table 2**

| Buffer formulation | | | | | | | |
|---|---|---|---|---|---|---|---|
| | Buffer-1 | Buffer-2 | Buffer-3 | Buffer-4 | Buffer- | 5 Buffer-6 | Standard Buffer |
| DI-Water | 1000.000 | 1000.000 | 1000.000 | 1000.000 | 1000.000 | 1000.000 | 1000.000 |
| NaCl | 8.300 | 8.300 | 8.300 | 8.300 | 8.300 | 8.300 | 8.300 |
| NaH₂PO₄.2H₂O | 0.530 | 0.530 | 0.530 | 0.530 | 0.530 | 0.530 | 0.530 |
| Na₂HPO₄ | 2.380 | 2.380 | 2.380 | 2.380 | 2.380 | 2.380 | 2.380 |
| PVP360K | 0.600 | | 0.600 | | 0.600 | 0.600 | |
| PVP8K | | | | 0.600 | | | |
| PVA-PVAc-1 | | 0.200 | 0.200 | | | | |
| PVA-PVAc-2 | | | | 0.200 | 0.200 | | |
| PVA-PVAc-3 | | | | | | 0.200 | |

Annotation: The numerical unit in the Table 2 is gram (g). PVP360K is PVP with the molecular weight of 360,000 Da, PVP8K is PVP with the molecular weight of 8,000 Da. PVA-PVAc-1 is the PVA-PVAc copolymer with the molecular weight of 31,000 Da and PVA integrally accounting for 88%. PVA-PVAc-2 is the PVA-PVAc copolymer with the molecular weight of 205,000 Da and PVA integrally accounting for 88%. PVA-PVAc-3 is the PVA-PVAc copolymer with the molecular weight of 27,000 Da and PVA integrally accounting for 98%.

An implemented sample 1-2 (EX1-2): follow the procedure of the implemented sample 1-1 to prepare the implemented sample 1-2. After the aluminum foil and the pp cup are sealed after heat sealing, the wet lens is not sent to the autoclave for autoclaving but the wet lens is soaked in Buffer-1 in the pp cup with heat-sealed aluminum foil. In about 12 hours (including the sterilization process) after the wet lens is in contact with the buffer, an aluminum foil is torn apart, the buffer was extracted and tested, obtaining a pH value being 7.2 and an osmotic pressure range being 318 mOsm/ (kg H₂O); and the contact lens is taken out and placed in a standard buffer for balancing. One contact lens uses 10 ml of standard buffer. The buffer is replaced every 1 hour and repeat 3 times. Then the surface lubricity of the contact lens is measured.

An implemented sample 2-1 (EX2-1): manufacture a Hydrogel-2 dry lens, with the contact lens formulation shown in Table 1. A process of manufacturing the dry lens from the contact lens formulation is a known technology in the art, and the dry lens may be manufactured by a casting method. A curing mode of the contact lens may be photocuring or thermal curing, and in the implemented sample, photocuring is used. The dry lens is soaked in an aqueous solution containing 0.5wt% sodium carbonate for 20 min to make MAA in the dry lens rapidly ionized, so that the dry lens swells to increase the cleaning efficiency, then hydrated with RO water at room temperature (20 to 30 degrees Celsius) for 40 min, and residual monomer, crosslinker, initiator, and diluent in the dry lens after the curing reaction are cleaned to obtain a wet lens. After the wet lens is obtained, the wet lens is soaked in an aqueous solution containing 0.2 g/L PVA-PVAc copolymer for 120 min to make the PVA-PVAc copolymer enter the surface layer of the core lens body through mass transfer. The PVA-PVAc copolymer used herein is PVA-PVAc-1 with the molecular weight of 31,000 Da and PVA accounting for overall proportion of 88%, that is, partially hydrolyzed polyvinyl alcohol with a degree of hydrolysis being 88%. After the above steps are finished, the wet lens is moved out of the aqueous solution containing the PVA-PVAc copolymer and placed in a pp cup. The Buffer-1 is dropwise added into the pp cup, and the liquid level of the buffer must be higher than the wet lens to ensure that buffer covers all the wet lens. After the wet lens is covered with the buffer, using heat sealing to seal the aluminum foil with the pp cup, and the wet lens is sent into an autoclave for autoclaving. A sterilization condition is as follows: the temperature is continuously raised to 122 degrees Celsius, and the temperature of 122 degrees Celsius at two barometric pressures is maintained for 30 min, in order to obtain the contact lens, wherein the core lens body takes HEMA as a main component, the water content is about 55%, the shell layer is based on PVA-PVAc copolymer and PVP, and the core lens body and the PVP are connected through the PVA-PVAc copolymer. In about 12 hours (including the sterilization process) after the wet lens is in contact with the buffer, an aluminum foil is torn apart, the contact lens is taken out and placed in a standard buffer for balancing. One contact lens uses 10 ml of standard buffer. The buffer is replaced every 1 hour and repeat 3 times. Then the surface lubricity of the contact lens is scored.

An implemented sample 2-2 (EX2-2): follow the procedure of the implemented sample 2-1 to prepare the implemented sample 2-2. After the aluminum foil and the pp cup are sealed after heat sealing, the wet lens is not sent to the autoclave for autoclaving but the wet lens is soaked in Buffer-1 in the pp cup with heat-sealed aluminum foil. In about 12 hours (including the sterilization process) after the wet lens is in contact with the buffer, an aluminum foil is torn apart, the contact lens is taken out and placed in a standard buffer for balancing. One contact lens uses 10 ml of standard buffer, and the buffer is replaced every 1 hour and repeat 3 times. Then the surface lubricity of the contact lens is scored.

An implemented sample 3-1 (EX3-1): manufacture a Silicone Hydrogel dry lens, with the contact lens formulation shown in Table 1. A process of manufacturing the dry lens from the contact lens formulation is a known technology in the art, and the dry lens may be manufactured by a casting method. A curing mode of the contact lens may be photocuring or thermal curing, and in the implemented sample, photocuring is used. The dry lens is first soaked in an aqueous solution containing 50 wt% isopropanol (IPA) for 60 min to make the dry lens rapidly swell to increase the cleaning efficiency. Moreover, using IPA having high compatibility with the silicone containing components TRIS and MPDMS to ensure that TRIS and MPDMS that have not completely reacted during the curing reaction are washed away. Besides, residual other monomers, crosslinker, initiator, and diluent are also cleaned. Then, the lens is soaked in RO water at room temperature (20 to 30 degrees Celsius) 4 times, 30 min per time. The RO water is replaced by clean RO water every time to make sure that IPA is cleaned thoroughly, so as to obtain the wet lens. After the wet lens is obtained, the wet lens is soaked in an aqueous solution containing 0.2 g/L PVA-PVAc copolymer for 120 min to make the PVA-PVAc copolymer enter the surface layer of the core lens body through mass transfer. The PVA-PVAc copolymer used herein is PVA-PVAc-1 with the molecular weight of 31,000 Da and PVA accounting for overall proportion of 88%, that is, partially hydrolyzed polyvinyl alcohol with a degree of hydrolysis being 88%. After the above steps are finished, the wet lens is moved out of the aqueous solution containing the PVA-PVAc copolymer and placed in a pp cup. The Buffer-1 is dropwise added into the pp cup, and the liquid level of the buffer must be higher than the wet lens to ensure that buffer covers all the wet lens. After the wet lens is covered with the buffer, using heat sealing to seal the aluminum foil with the pp cup, and the wet lens is sent into an autoclave for autoclaving. A sterilization condition is as follows: the temperature is continuously raised to 122 degrees Celsius, and the temperature of 122 degrees Celsius at two barometric pressures is maintained for 30 min, in order to obtain the contact lens, wherein the core lens body is a silicone hydrogel, the shell layer is based on PVA-PVAc copolymer and PVP, and the core lens body and the PVP are connected through the PVA-PVAc copolymer. In about 12 hours (including the sterilization process) after the wet lens is in contact with the buffer, an aluminum foil is torn apart, the buffer was extracted and tested, obtaining a pH value being 7.2 and an osmotic pressure range being 307 mOsm/ (kg H₂O); and the contact lens is taken out and placed in a standard buffer for balancing. One contact lens uses 10 ml of standard buffer. The buffer is replaced every 1 hour and repeat 3 times. Then the surface lubricity of the contact lens is scored.

An implemented sample 3-2 (EX3-2): follow the procedure of the implemented sample 3-1 to prepare the implemented sample 2-2. After the aluminum foil and the pp cup are sealed after heat sealing, the wet lens is not sent to the autoclave for autoclaving but the wet lens is soaked in Buffer-1 in the pp cup with heat-sealed aluminum foil. In about 12 hours (including the sterilization process) after the wet lens is in contact with the buffer, an aluminum foil is torn apart, the contact lens is taken out and placed in a standard buffer for balancing. One contact lens uses 10 ml of standard buffer, and the buffer is replaced every 1 hour and repeat 3 times. Then the surface lubricity of the contact lens is scored.

An implemented sample 4-1 (EX4-1): manufacture a Hydrogel-2 dry lens, with the contact lens formulation shown in Table 1. A process of manufacturing the dry lens from the contact lens formulation is a known technology in the art, and the dry lens may be manufactured by a casting method. A curing mode of the contact lens may be photocuring or thermal curing, and in the implemented sample, photocuring is used. The dry lens is soaked in an aqueous solution containing 0.5wt% sodium carbonate for 20 min to make MAA in the dry lens rapidly ionized, so that the dry lens swells to increase the cleaning efficiency, and then is hydrated with RO water at room temperature (20 to 30 degrees Celsius) for 40 min, and residual monomer, crosslinker, initiator, and diluent in the dry lens after the curing reaction are cleaned to obtain a wet lens. After the wet lens is obtained, the wet lens is placed in a pp cup. The Buffer-3 is dropwise added into the pp cup, and the liquid level of the buffer must be higher than the wet lens to ensure that buffer covers all the wet lens. After the wet lens is covered with the buffer, using heat sealing to seal the aluminum foil with the pp cup, and the set lens is sent into an autoclave for autoclaving. A sterilization condition is as follows: the temperature is continuously raised to 122 degrees Celsius, and the temperature of 122 degrees Celsius at two barometric pressures is maintained for 30 min, in order to obtain the contact lens, wherein the core lens body takes HEMA as a main component, the water content is about 55%, the shell layer is based on PVA-PVAc copolymer and PVP, and the core lens body and the PVP are connected through the PVA-PVAc copolymer. In about 12 hours (including the sterilization process) after the wet lens is in contact with the buffer, an aluminum foil is torn apart, the buffer was extracted and tested, obtaining a pH value being 7.3 and an osmotic pressure range being 321 mOsm/ (kg H₂O); and the contact lens is taken out and placed in a standard buffer for balancing. One contact lens uses 10 ml of standard buffer. The buffer is replaced every 1 hour and repeat 3 times. Then the surface lubricity of the contact lens is scored. A formulation of the Buffer-3 is shown in Table 2.

An implemented sample 4-2 (EX4-2): manufacture a Hydrogel-2 dry lens, with the contact lens formulation shown in Table 1. A process of manufacturing the dry lens from the contact lens formulation is a known technology in the art, and the dry lens may be manufactured by a casting method. A curing mode of the contact lens may be photocuring or thermal curing, and in the implemented sample, photocuring is used. The dry lens is soaked in an aqueous solution containing 0.5wt% sodium carbonate for 20 min to make MAA in the dry lens rapidly ionized, so that the dry lens swells to increase the cleaning efficiency, and then is hydrated with RO water at room temperature (20 to-30 degrees Celsius) for 40 min, and residual monomer, crosslinker, initiator, and diluent in the dry lens after the curing reaction are cleaned to obtain a wet lens. After the wet lens is obtained, the wet lens is soaked in an aqueous solution containing 0.6 g/L PVP for 120 min to make the PVP enter the surface layer of the core lens body through mass transfer. The PVP used herein is PVP360 with the molecular weight of 360,000 Da. After the above steps are finished, the wet lens is moved out of the aqueous solution containing the PVP and placed in a pp cup. The Buffer-2 is dropwise added into the pp cup, and the liquid level of the buffer must be higher than the wet lens to ensure that buffer covers all the wet lens. After the wet lens is covered with the buffer, using heat sealing to seal the aluminum foil with the pp cup, and the wet lens is sent into an autoclave for autoclaving. A sterilization condition is as follows: the temperature is continuously raised to 122 degrees Celsius, and the temperature of 122 degrees Celsius at two barometric pressures is maintained for 30 min. In about 12 hours (including the sterilization process) after the wet lens is in contact with the buffer, an aluminum foil is torn apart, the buffer was extracted and tested, obtaining a pH value being 7.2 and an osmotic pressure range being 320 mOsm/ (kg H₂O); and the contact lens is taken out and placed in a standard buffer for balancing. One contact lens uses 10 ml of standard buffer. The buffer is replaced every 1 hour and repeat 3 times. Then the surface lubricity of the contact lens is scored. A formulation of the Buffer-2 is shown in Table 2.

Implemented sample 5-1 (EX5-1): follow the procedure of the implemented sample 4-1 to prepare the implemented sample 5-1, and the PVP in the buffer is replaced with Copolymer 845 (the molecular weight of the polymer is around 1,000,000 Da). In about 12 hours (including the sterilization process) after the wet lens is in contact with the buffer, an aluminum foil is torn apart, the buffer was extracted and tested, obtaining a pH value being 7.4 and an osmotic pressure range being 288 mOsm/ (kg H₂O); and the contact lens is taken out and placed in a standard buffer for balancing. One contact lens uses 10 ml of standard buffer, and the buffer is replaced every 1 hour and repeat 3 times. Then the surface lubricity of the contact lens is scored.

An implemented sample 5-2 (EX5-2): manufacture a Hydrogel-2 dry lens, with the contact lens formulation shown in Table 1. A process of manufacturing the dry lens from the contact lens formulation is a known technology in the art, and the dry lens may be manufactured by a casting method. A curing mode of the contact lens may be photocuring or thermal curing, and in the implemented sample, photocuring is used. The dry lens is soaked in an aqueous solution containing 0.5wt% sodium carbonate for 20 min to make MAA in the dry lens rapidly ionized, so that the dry lens swells to increase the cleaning efficiency, and then is hydrated with RO water at room temperature (20 to 30 degrees Celsius) for 40 min, and residual monomer, crosslinker, initiator, and diluent in the dry lens after the curing reaction are cleaned to obtain a wet lens. After the wet lens is obtained, the wet lens is placed in a pp cup, the Buffer-2 is dropwise added into the pp cup, and the liquid level of the buffer must be higher than the wet lens to ensure that buffer covers all the wet lens. After the wet lens is covered with the buffer, using heat sealing to seal the aluminum foil with the pp cup, and the wet lens is sent into an autoclave for autoclaving. A sterilization condition is as follows: the temperature is continuously raised to 122 degrees Celsius, and the temperature of 122 degrees Celsius at two barometric pressures is maintained for 30 min. After the above sterilization is finished, the aluminum foil is torn apart, the contact lens is taken out and then placed in a new pp cup, the Buffer-1 is dropwise added into the pp cup, and the liquid level of the buffer must be higher than the wet lens to ensure that buffer covers all the wet lens. After the wet lens is covered with the buffer, using heat sealing to seal the aluminum foil with the pp cup, and the wet lens is sent into an autoclave for the second autoclaving. A sterilization condition is as follows: the temperature is continuously raised to 122 degrees Celsius, and the temperature of 122 degrees Celsius at two barometric pressures is maintained for 30 min. After sterilization, the aluminum foil is torn apart, the buffer was extracted and tested, obtaining a pH value being 7.2 and an osmotic pressure range being 315 mOsm/ (kg H₂O); and the contact lens is taken out and placed in a standard buffer for balancing. One contact lens uses 10 ml of standard buffer. The buffer is replaced every 1 hour and repeat 3 times. Then the surface lubricity of the contact lens is scored.

Implemented sample 6-1 (EX6-1): follow the procedure of the implemented sample 4-1 to prepare the implemented sample 6-1, and the buffer is replaced by a Buffer-4, wherein the formulation of the Buffer-4 is shown in Table 2. After sterilization is completed, in about 12 hours (including the sterilization process) after the wet lens is in contact with the buffer, an aluminum foil is torn apart, the buffer was extracted and tested, obtaining a pH value being 7.2 and an osmotic pressure range being 307 mOsm/ (kg H₂O); and the contact lens is taken out and placed in a standard buffer for balancing. One contact lens uses 10 ml of standard buffer, and the buffer is replaced every 1 hour and repeat 3 times. Then the surface lubricity of the contact lens is scored.

Implemented sample 7-1 (EX7-1): follow the procedure of the implemented sample 4-1 to prepare the implemented sample 7-1, and the Buffer-3 is replaced by a Buffer-5, wherein the formulation of the Buffer-5 is shown in Table 2. After sterilization is completed, in about 12 hours (including the sterilization process) after the wet lens is in contact with the buffer, an aluminum foil is torn apart, the buffer was extracted and tested, obtaining a pH value being 7.3 and an osmotic pressure range being 311 mOsm/ (kg H₂O); and the contact lens is taken out and placed in a standard buffer for balancing. One contact lens uses 10 ml of standard buffer, and the buffer is replaced every 1 hour and repeat 3 times. Then the surface lubricity of the contact lens is scored.

Implemented sample 8-1 (EX8-1): follow the procedure of the implemented sample 4-1 to prepare the implemented sample 8-1, and the Buffer-3 is replaced by a Buffer-6, wherein the formulation of the Buffer-6 is shown in Table 2. After sterilization is completed, in about 12 hours (including the sterilization process) after the wet lens is in contact with the buffer, an aluminum foil is torn apart, the buffer was extracted and tested, obtaining a pH value being 7.2 and an osmotic pressure range being 313 mOsm/ (kg H₂O); and the contact lens is taken out and placed in a standard buffer for balancing. One contact lens uses 10 ml of standard buffer, and the buffer is replaced every 1 hour and repeat 3 times. Then the surface lubricity of the contact lens is scored.

An implemented sample 9-1 (EX9-1): manufacture a Hydrogel-2 dry lens, with the contact lens formulation shown in Table 1. A process of manufacturing the dry lens from the contact lens formulation is a known technology in the art, and the dry lens may be manufactured by a casting method. A curing mode of the contact lens may be photocuring or thermal curing, and in the implemented sample, photocuring is used. The dry lens is soaked in an aqueous solution containing 0.5wt% sodium carbonate for 20 min to make MAA in the dry lens rapidly ionized, so that the dry lens swells to increase the cleaning efficiency. The lens is then hydrated with RO water at room temperature (20 to 30 degrees Celsius) for 40 min, and residual monomer, crosslinker, initiator, and diluent in the dry lens after the curing reaction are cleaned to obtain a wet lens. After the wet lens is obtained, the wet lens is then soaked in an aqueous solution containing 0.2 g/L PVA (the molecular weight is 145,000 Da, and the degree of hydrolysis is 99%) to be heated for 120 min at 70±5 degrees Celsius. Then the wet lens is soaked in an aqueous solution containing 0.6 g/L PVP (the molecular weight is 360,000 Da) to be heated for 120 min at 70±5 degrees Celsius to obtain the wet lens. The wet lens is placed in a pp cup and a standard buffer is dropwise added into the pp cup. The liquid level of the buffer must be higher than the wet lens to ensure that buffer covers all the wet lens. After the wet lens is covered with the buffer, using heat sealing to seal the aluminum foil with the pp cup, and the wet lens is sent into an autoclave for autoclaving. A sterilization condition is as follows: the temperature is continuously raised to 122 degrees Celsius, and the temperature of 122 degrees Celsius at two barometric pressures is maintained for 30 min. In about 12 hours (including the sterilization process) after the wet lens is in contact with the buffer, an aluminum foil is torn apart, the buffer was extracted and tested, obtaining a pH value being 7.4 and an osmotic pressure range being 320 mOsm/ (kg H₂O); and the contact lens is taken out and placed in a standard buffer for balancing. One contact lens uses 10 ml of standard buffer, and the buffer is replaced every 1 hour and repeat 3 times. Then the surface lubricity of the contact lens is scored. A formulation of the standard buffer is shown in Table 2.

An implemented sample 6-2 (EX6-2): follow the procedure of the implemented sample 9-1 to prepare the implemented sample 6-2. After the aluminum foil and the pp cup are sealed after heat sealing, the wet lens is not sent to the autoclave for autoclaving but the wet lens is soaked in the standard buffer in the pp cup heat-sealed with the aluminum foil. In about 12 hours (including the sterilization process) after the wet lens is in contact with the buffer, an aluminum foil is torn apart, the buffer was extracted and tested, obtaining a pH value being 7.2 and an osmotic pressure range being 310 mOsm/ (kg H₂O); and the contact lens is taken out and placed in a standard buffer for balancing. One contact lens uses 10 ml of standard buffer, and the buffer is replaced every 1 hour and repeat 3 times. Then the surface lubricity of the contact lens is scored.

Implemented sample 7-2 (EX7-2): follow the procedure of the implemented sample 4-1 to prepare the implemented sample 7-2, and the Buffer-3 is replaced by a Buffer-1, wherein the formulation of the Buffer-1 is shown in Table 2. After sterilization is completed, in about 12 hours (including the sterilization process) after the wet lens is in contact with the buffer, an aluminum foil is torn apart, the buffer was extracted and tested, obtaining a pH value being 7.2 and an osmotic pressure range being 320 mOsm/ (kg H₂O); and the contact lens is taken out and placed in a standard buffer for balancing. One contact lens uses 10 ml of standard buffer, and the buffer is replaced every 1 hour and repeat 3 times. Then the surface lubricity of the contact lens is scored.

An implemented sample 8-2 (EX8-2): follow the procedure of the implemented sample 7-2 to prepare the implemented sample 8-2. Before the surface lubricity of the contact lens is measured, the aluminum foil is torn apart first, the contact lens is taken out and placed in the standard buffer for balancing. One contact lens uses 10 ml of standard buffer, the buffer is replaced every 1 hour and repeat 3 times. Then the surface lubricity of the contact lens is scored.

Implemented sample 9-2 (EX9-2): follow the procedure of the implemented sample 4-1 to prepare the implemented sample 9-2, and the Buffer-3 is replaced by a Buffer-2, wherein the formulation of the Buffer-2 is shown in Table 2. After sterilization is completed, in about 12 hours (including the sterilization process) after the wet lens is in contact with the buffer, an aluminum foil is torn apart, the contact lens is taken out and placed in a standard buffer for balancing. One contact lens uses 10 ml of standard buffer, and the buffer is replaced every 1 hour and repeat 3 times. Then the surface lubricity of the contact lens is scored.

Surface lubricity score of the contact lens. The surface lubricity of the contact lens is differentiated with 1-4 score. The higher score represents higher surface lubricity of the contact lens. A score of 1 represents no lubricity when the contact lens is rubbed with fingers. A score of 2 represents little lubricity when the contact lens is rubbed with fingers. The lubricity of the contact lens with score of 2 is equivalent to the lubricity of a commercially available contact lens product senofilcon A contact lens. A score of 3 represents appropriate lubricity of the contact lens. A score of 4 represents quite smooth texture of the contact lens. The lubricity of the contact lens with a score of 4 is equivalent to the lubricity of a commercially available contact lens product delefilcon A contact lens. It is to be noted that though the experience of wearing the delefilcon A contact lens is very good among some users, however, the contact lens is too smooth to remove the contact lens from eyes after use, which affects the purchase intention of the users. Therefore, the higher lubricity of the contact lens is not always the better. In evaluation of the present invention, the most appropriate lubricity is a score of 3.

A test mode of the surface lubricity score is as follows: 5 testers are selected. Each tester will rub the senofilcon A contact lens and the delefilcon A contact lens synchronously. The lubricity of rubbing feeling of the senofilcon A contact lens is calibrated as 2 score, and the lubricity of rubbing feeling of the delefilcon A contact lens is calibrated as 4 score. If 4 in the 5 testers think that the lubricity of the test sample is obviously lower than that of the senofilcon A contact lens, the lubricity score of the test sample will be recorded as 1 score. If only 3 testers think that the lubricity of the test sample is lower than that of the senofilcon A contact lens and 2 testers think that the lubricity of the test sample is not significantly different from that of the senofilcon A contact lens, the lubricity is recorded as 1-2 score. If more than 4 testers think that the lubricity of the test sample is equivalent to that of the senofilcon A contact lens, the lubricity is recorded as 2 score. If more than 3 testers think that the lubricity of the test sample is obviously higher than that of the senofilcon A contact lens and the other 2 testers think that the lubricity of the test sample is equivalent to that of the senofilcon A contact lens, the lubricity is recorded as 2-3 score. If more than 4 testers think that the lubricity is obviously higher than that of the senofilcon A contact lens, the lubricity is recorded as 3 score.

The surface lubricity score of the implemented sample is shown in Table 3.

**Table 3**

| Implemented sample | Surface lubricity score |
|---|---|
| EX1-1 | 3 |
| EX2-1 | 3 |
| EX3-1 | 3 |
| EX1-2 | 1 |
| EX2-2 | 1 |
| EX3-2 | 1 |
| EX4-1 | 3 |
| EX5-1 | 3 |
| EX4-2 | 2 |
| EX5-2 | 2-3 |
| EX6-1 | 2 |
| EX7-1 | 3 |
| EX8-1 | 2-3 |
| EX9-1 | 2-3 |
| EX6-2 | 1 |
| EX7-2 | 2 |
| EX8-2 | 1 |
| EX9-2 | 1 |

It can be seen from data in Table 3 that according to the technical solution (the implemented samples EX1-1, 2-1, 3-1, 4-1, 5-1, 6-1, 7-1, 8-1, and 9-1) provided in the present invention, the surface lubricity can be effectively enhanced, and the surface lubricity is controlled in an appropriate range. The main difference between the implemented sample 1-1 (EX1-1) and the implemented sample 1-2 (EX1-2) lies in that whether there is sterilization. The implemented sample 1-1 with sterilization has the higher surface lubricity, with the score of 3. Similarly, the surface lubricity score of the implemented sample 2-1 (EX2-1) is higher than that of the implemented sample 2-2 (EX2-2). The surface lubricity score of the implemented sample 3-1 (EX3-1) is higher than that of the implemented sample 3-2 (EX3-2) due to the same reason compared with the implemented sample 1-1 (EX1-1), which is not repeatedly described herein. The implemented sample 4-1 (EX4-1) is similar to the implemented sample 4-2 (EX4-2). The main difference therebetween lies in that the time when the first polymer and the second polymer are in contact with the core lens body is different. In the implemented sample 4-1 (EX4-1), the core lens body is soaked in the buffer containing both the first and second polymers, so that the first polymer can effectively form the IPN with the surface structure of the core lens body, which, therefore, affects the surface lubricity of the final contact lens. The main difference between the implemented sample 5-1 (EX5-1) and the implemented sample 5-2 (EX5-2) lies in that the implemented sample 5-2 is sterilized twice: it is first soaked in the buffer-2 containing the PVA for first sterilization and then soaked in the buffer-1 containing the PVP for second sterilization. Viewed from the surface lubricity score, it can be known that the score of the implemented sample 5-1 is higher than that of the implemented sample 5-2. The reason may be as follows: because when the implemented sample 5-2 is subjected to the first sterilization, strong hydrogen bonds and crosslinking reaction are formed among part of polyvinyl alcohol in the first polymer, which hinders formation of the shell layer by the second polymer and the first polymer during second sterilization. Therefore, the shell layer may be unstable, which results in the relatively low surface lubricity score. Compared with the implemented sample 6-2(EX6-2), the surface lubricity score of the implemented sample 9-1(EX9-1) is higher as the implemented sample 9-1 is subjected to sterilization. The implemented sample 4-1(EX4-1) is similar to the implemented sample 7-2(EX7-2), and the main difference therebetween lies in that besides the second polymer in the buffer-3 where the implemented sample 4-1 is soaked, the buffer further contains the first polymer. Therefore, helped by the first polymer, the second polymer is capable of stably existing on the surface of the core lens body. Therefore, the surface lubricity score of the implemented sample 4-1 is higher. Similarly, the surface lubricity score of the implemented sample 4-1 (EX4-1) is higher than that of the implemented sample 8-2 (EX8-2), which is not repeatedly described herein. Compared with the buffer where the implemented sample 9-2 (EX9-2) is soaked, besides the first polymer in the buffer-3 where the implemented sample 4-1 (EX4-1) is soaked, the buffer-3 further has the second polymer, which contributes to forming the shell layer and increasing the surface lubricity of the contact lens. Therefore, the implemented sample 4-1 has the higher surface lubricity score than that of the implemented sample 9-2.

The contact lenses of the implemented samples 4-1 and 7-2 are cleaned 3 times with RO water to remove components (including buffer salts NaCl, NaH₂PO₄, and Na₂HPO₄) that can be removed by RO water on the surfaces of the contact lenses. Then the contact lenses are dried at 105°C for at least 8 hours and water of the contact lenses is removed to obtain dry lenses. The surface components of the dry lenses are analyzed with element analysis function of the scanning electron microscope (Energy-dispersive X-ray spectroscopy with Scanning Electron Microscope, SEM-EDX). Element analysis data shows that the surface of the dry lens treated in the implemented sample 4-1 contains 4.39% nitrogen element, indicating that even if the dry lens is rinsed with RO water, the second polymer (PVP) still stably exists on the surface of the core lens body. However, nitrogen element cannot be detected on the surface of the dry lens treated in the implemented sample 7-2, indicating that the second polymer (PVP) that originally existing on the surface of the core lens body has been washed away after being rinsed with RO water. Therefore, the SEM-EDX analytical mode can further indicate that the core lens body of the contact lens in an implemented sample of the present invention is covered by the shell layer containing the second polymer (PVP).

Implemented samples 10~15 (EX10~EX15): follow the procedure of the implemented sample 4-1 to prepare the implemented samples 10~15, and the Buffer-3 is replaced with Buffer-7~Buffer-12, wherein the formulations of the buffers are recorded in Table 4. That is, the implemented sample 10 uses Buffer-7 instead of Buffer-3; the implemented sample 11 uses Buffer-8 instead of Buffer-3; the implemented sample 12 uses Buffer-9 instead of Buffer-3; the implemented sample 13 uses Buffer-10 instead of Buffer-3; the implemented sample 14 uses Buffer-11 instead of Buffer-3; the implemented sample 15 uses Buffer-12 instead of Buffer-3. After sterilization is completed, in about 12 hours (including the sterilization process) after the wet lens is in contact with the buffer, an aluminum foil is torn apart, the buffer was extracted and tested, obtaining a pH value and an osmotic pressure. The implemented samples 10~15 have pH values ranging from 6.9 to 7.6, and osmotic pressures ranging from 288 to 337 mOsm/ (kg • H₂O). The implemented samples 10~15 were evaluated in the same manner as in the implemented sample 4-1 for evaluating the surface lubricity, and the surface lubricity score is shown in Table 5.

**Table 4**

| Buffer Formulation | | | | | | |
|---|---|---|---|---|---|---|
| | Buffer-7 | Buffer-8 | Buffer-9 | Buffer-10 | Buffer-11 | Buffer-12 |
| DI-Water | 1000.000 | 1000.000 | 1000.000 | 1000.000 | 1000.000 | 1000.000 |
| NaCl | 8.300 | 8.300 | 1.800 | 1.800 | 1.750 | 1.750 |
| NaH₂PO₄.2H₂O | 0.530 | 0.530 | 4.200 | 4.200 | 4.400 | 4.400 |
| Na₂HPO₄ | 2.380 | 2.380 | 16.000 | 16.000 | 16.800 | 16.800 |
| PVP360K | 0.200 | | 0.600 | | 0.800 | 0.640 |
| PVP8K | | 1.500 | | 1.500 | | |
| PVA-PVAc-1 | | | | | | 0.080 |
| PVA-PVAc-2 | 0.400 | 0.100 | 0.300 | 0.150 | | |
| PVA-PVAc-3 | | | | | 0.200 | |

Annotation: The numerical unit in the Table 4 is gram (g). PVP360K is PVP with the molecular weight of 360,000 Da, PVP8K is PVP with the molecular weight of 8,000 Da. PVA-PVAc-1 is the PVA-PVAc copolymer with the molecular weight of 31,000 Da and PVA integrally accounting for 88%. PVA-PVAc-2 is the PVA-PVAc copolymer with the molecular weight of 205,000 Da and PVA integrally accounting for 88%. PVA-PVAc-3 is the PVA-PVAc copolymer with the molecular weight of 27,000 Da and PVA integrally accounting for 98%.

**Table 5**

| Implemented sample | Surface lubricity score |
|---|---|
| EX10 | 2-3 |
| EX11 | 2-3 |
| EX12 | 3 |
| EX13 | 2-3 |
| EX14 | 3 |
| EX15 | 3 |

Implemented sample 16 (EX16): follow the procedure of the implemented sample 1-1 to prepare the implemented sample 16. However, after removed from the PVA-PVAc copolymer aqueous solution, the wet lens is not placed in a pp cup to contact the buffer, but is placed in a PVP360 aqueous solution with a concentration of 0.6 g/L and heated at 80 degrees Celsius for 2 hours. Then, the wet lens is placed in a pp cup, and the standard buffer is dropwise added into the pp cup, and the liquid level of the buffer must be higher than the wet lens to ensure that buffer covers all the wet lens. After the wet lens is covered with the buffer, using heat sealing to seal the aluminum foil with the pp cup, and the wet lens is sent into an autoclave for autoclaving. A sterilization condition is as follows: the temperature is continuously raised to 122 degrees Celsius, and the temperature of 122 degrees Celsius at two barometric pressures is maintained for 30 minutes.

Implemented sample 17 (EX17): follow the procedure of the implemented sample 2-1 to prepare the implemented sample 17. However, after removed from the PVA-PVAc copolymer aqueous solution, the wet lens is not placed in a pp cup to contact the buffer, but is placed in a PVP360 aqueous solution with a concentration of 0.6 g/L and heated at 80 degrees Celsius for 2 hours. Then, the wet lens is placed in a pp cup, and the standard buffer is dropwise added into the pp cup, and the liquid level of the buffer must be higher than the wet lens to ensure that buffer covers all the wet lens. After the wet lens is covered with the buffer, using heat sealing to seal the aluminum foil with the pp cup, and the wet lens is sent into an autoclave for autoclaving. A sterilization condition is as follows: the temperature is continuously raised to 122 degrees Celsius, and the temperature of 122 degrees Celsius at two barometric pressures is maintained for 30 minutes.

Implemented sample 18 (EX18): follow the procedure of the implemented sample 17 to prepare the implemented sample 18, sodium carbonate used in the hydration process is replaced with sodium tetraborate, and the rest of the production process remains the same.

Implemented sample 19 (EX19): follow the procedure of the implemented sample 16 to prepare the implemented sample 19, buffer is replaced with Buffer-3, and the rest of the production process remains the same.

Implemented sample 20 (EX20): follow the procedure of the implemented sample 17 to prepare the implemented sample 20, buffer is replaced with Buffer-3, and the rest of the production process remains the same.

Implemented sample 21 (EX21): follow the procedure of the implemented sample 2-1 to prepare the implemented sample 21, buffer is replaced with Buffer-3, and the rest of the production process remains the same.

Evaluation of the surface lubricity of implemented samples 16~21: In about 12 hours (including the sterilization process) after the wet lens is in contact with the buffer, an aluminum foil is torn apart, the contact lens is taken out and placed in a standard buffer for balancing. One contact lens uses 10 ml of standard buffer, and the buffer is replaced every 1 hour and repeat 3 times. Then the surface lubricity of the contact lens is scored and the scores are summarized in Table 6. From the comparison of EX16, EX17 with EX1-1 and EX2-1(see Table 3), it could be seen that the surface lubricity score of the contact lens is lower only when the contact lens is in contact with the first polymer and the second polymer during the hydration process. It is speculated that the temperature during sterilization (122 degrees Celsius) is higher than the hydration temperature (from 20 degrees Celsius to 80 degrees Celsius), which is more conducive to the formation of the shell layer of the contact lens. From the comparison between EX19 and EX20, the surface lubricity score of EX20 is higher. It is speculated that this is because the proportion of MAA in the core lens body is higher, which is beneficial for the first polymer to be adsorbed to the surface of the wet lens, so the shell layer is formed more completely. EX20 has a higher surface lubricity score than EX4-1 (see Table 3), presumably because the core lens body is in contact with the first polymer and the second polymer during the sterilization process and also during the hydration process.

**Table 6**

| Implemented sample | Surface lubricity score |
|---|---|
| EX16 | 2-3 |
| EX17 | 2-3 |
| EX18 | 3 |
| EX19 | 3 |
| EX20 | 3-4 |
| EX21 | 3 |

It can be verified from the above data analysis and description that the technical solution provided by the present invention can effectively make the contact lens have the appropriate surface lubricity, which contributes to increasing the comfort of the contact lens in wearing.

While the invention has been described in terms of what is presently considered to be the most practical and preferred embodiments, it is to be understood that the invention needs not be limited to the disclosed embodiment. On the contrary, it is intended to cover various modifications and similar arrangements included within the spirit and scope of the appended claims which are to be accorded with the broadest interpretation so as to encompass all such modifications and similar structures.

## Claims

1. A contact lens packaging solution (200), suitable for soaking a core lens body (110), and performing a sterilization step when the core lens body (110) is soaked in the contact lens packaging solution (200), **characterized in that** the contact lens packaging solution (200) comprises a first polymer and a second polymer, the first polymer comprises polyvinyl alcohol, and the second polymer comprises polyvinylpyrrolidone.

2. The contact lens packaging solution (200) according to claim 1, **characterized in that** the first polymer is partially hydrolyzed polyvinyl alcohol with a degree of hydrolysis of 70%~99%.

3. The contact lens packaging solution (200) according to claim 1 or 2, **characterized in that** the first polymer is partially hydrolyzed polyvinyl alcohol with a degree of hydrolysis of 80%~98%.

4. The contact lens packaging solution (200) according to any one of claims 1-3, **characterized in that** the first polymer is partially hydrolyzed polyvinyl alcohol with a degree of hydrolysis of 88%~95%.

5. The contact lens packaging solution (200) according to any one of claims 1-4, **characterized in that** the molecular weight of the second polymer is greater than or equal to 8,000 Da.

6. The contact lens packaging solution (200) according to any one of claims 1-5, **characterized in that** the molecular weight of the second polymer is greater than or equal to 160,000 Da.

7. The contact lens packaging solution (200) according to any one of claims 1-6, **characterized in that** the molecular weight of the second polymer is greater than or equal to 360,000 Da.

8. The contact lens packaging solution (200) according to any one of claims 1-7, **characterized in that** the second polymer is a copolymer comprising polyvinylpyrrolidone.

9. The contact lens packaging solution (200) according to claim 8, **characterized in that** the second polymer is a copolymer of vinylpyrrolidone and dimethylaminoethyl methacrylate.

10. The contact lens packaging solution (200) according to claim 9, **characterized in that** the molecular weight of the second polymer is greater than or equal to 100,000 Da, preferably greater than or equal to 1,000,000 Da.

11. The contact lens packaging solution (200) according to any one of claims 1-10, **characterized in that** the relative proportion by weight percentage of the first polymer to the second polymer is between 2:1 and 1:15.

12. The contact lens packaging solution (200) according to any one of claims 1-11, **characterized in that** the relative proportion by weight percentage of the first polymer to the second polymer is between 1:2 and 1:10.

13. The contact lens packaging solution (200) according to any one of claims 1-12, **characterized in that** the relative proportion by weight percentage of the first polymer to the second polymer is between 1:4 and 1:8.

14. The contact lens packaging solution (200) according to any one of claims 1-13, **characterized in that** the pH value of the contact lens packaging solution (200) ranges from 6.0 to 8.0, and the osmotic pressure of the contact lens packaging solution (200) ranges from 200 to 400 mOsm/ (kg H₂O).

15. A method for manufacturing a contact lens (100), **characterized in that** comprising:
when soaking a core lens body (110) in the contact lens packaging solution (200) as described in any one of claims 1-14; and
performing a sterilization step, and then obtaining a contact lens (100);
wherein the contact lens (100) comprises the core lens body (110) and a shell layer (120), and the shell layer (120) covers the core lens body (110); wherein the shell layer (120) comprises the first polymer (121) and the second polymer (122).
